# EUROPÄISCHE PATENTSCHRIFT

(11) **EP 0 625 968 B1**
(45) Veröffentlichungstag und Bekanntmachung des Hinweises auf die Patenterteilung: **16.04.1997**
(21) Anmeldenummer: 93917386.0
(22) Anmeldetag: 04.02.1993
(51) Int. Cl.: C07D 209/04, C07D 209/30

(54) **SYNTHESE VON INDOLEN DURCH DEHYDRIERUNG VON INDOLINEN**
SYNTHESIS OF INDOLES BY DEHYDRATION OF INDOLINES
SYNTHESE D'INDOLES PAR DESHYDRATATION D'INDOLINES

(30) Priorität: 12.02.1992 DE 4204089
(43) Veröffentlichungstag der Anmeldung: 30.11.1994
(73) Patentinhaber: Henkel Kommanditgesellschaft auf Aktien, 40191 Düsseldorf (DE)
(72) Erfinder: KNÜBEL, Georg, D-4000 Düsseldorf 1 (DE); MICHEL, Roswitha, D-4000 Düsseldorf 13 (DE); ROSE, David, D-4010 Hilden (DE)
(86) Internationale Anmeldenummer: EP9300261
(87) Internationale Veröffentlichungsnummer: WO9316046

(56) Entgegenhaltungen:
- RUSSIAN CHEMICAL REVIEWS Bd. 36, Nr. 10, 1967, Seiten 753 - 771 M.N. PREOBRASHENSKAYA 'Synthesis of substituted indoles via indolines'
- BULL. SOC. CHIM. FR. 1966, Seiten 1335 - 1342 M. JULIA, H. GASTON-BRETON 'Recherches en série indolique. XVII. - Préparation de quelque indolines, indoles et tryptamines oxygénés en position -4 ou -6 par cyclisation "arynique"'
- J. CHEM. SOC. (C) 1967, Seiten 1424 - 1427 S.N. MISHRA, G.A. SWAN 'Studies Related to the Chemistry of Melanins. Part III. Synthesis of 5,6-Dihydroxyindoline'
- TETRAHEDRON LETT. Nr. 10, 1970, Seiten 723 - 726 T. KAMETANI ET AL. 'A Novel Formation of the Indole Derivatives by Phenolic Oxidative Coupling'
- BIOCHEM. Bd. 7, Nr. 5, 1968, Seiten 1777 - 1786 M. WILCHEK ET AL. 'The Nonenzymatic Conversion of Tyrosine into Mono- and Dihydroxyindoles'
- J. CHEM. SOC. 1950, Seiten 1276 - 1282 J. HARLEY-MASON 'The Chemistry of Adrenochrome and its Derivatives'

## Beschreibung

Die Erfindung betrifft ein Verfahren zur Synthese von 5-6-Dihydroxyindolen durch Dehydrierung, vorzugsweise katalytische Dehydrierung, von Indolinen.

Indole sind im Bereich der Pharmazie, der Kosmetik und der Färberei von großer Bedeutung.
Das wichtigste Verfahren zur Synthese von Indolderivaten ist die Fischer'sche Indolsynthese. Hierbei wird, ausgehend vom Phenylhydrazon einr Ketoverbindung durch Diaza-Cope-Umlagerung und anschließende Ammoniak-Abspaltung das entsprechende Indolderivat gebildet. Dieses Verfahren ist jedoch nicht uneingeschränkt anwendbar, vor allem dann nicht, wenn sich am aromatischen 6-Ring zusätzliche Substituenten befinden.

Besonders schwierig gestaltet sich die Synthese von 5,6-Dihydroxyindolen, da 5,6-Dihydroxyindole aufgrund ihrer inhärent doppelten Hydrochinonstruktur sehr oxidationsempfindlich sind. Zu ihrer Synthese bedarf es anderer Verfahren, die sich in 3 Gruppen einteilen lassen:
- Bildung des 5,6-Dihydroxyindolgerüsts druch reduktive Cyclisierung von geeigneten Nitrobenzolvorläufern (B. P. Murphy, T. M. Schultz, J. Org. Chem. 50 (1985)2790),
- Bildung des 5,6-Dihydroxyindolgerüsts durch oxidative Cyclisierung von Katecholaminen (J. D. Bu'Lock, J. Harley-Mason, J. Chem. Soc. (1951) 2248; J. Harley-Mason, J. D. Bu'Lock, Nature 166 (1950) 1036; K. Wakamatsu, S. Ito, Anal. Biochem. 170 (1988) 335; P. A. Wehrli, F. Pigott, U. Fischer, A. Kaiser, Helv. Chinm. Acta 55 (1972) 3057 und J. Harley-Mason, J. Chem. Soc. (1953) 200),
- Freisetzung von 5,6-Dihydroxyindolen aus 5,6-Dialkoxy- oder 5,6-Diacetoxyindolen (R. J. S. Beer, K. Clarke, H. E. Khorana, A. Robertson, J. Chem. Soc. (1948) 2223; H. Burton, J. A. Duffield, P. F. G. Praill, J. Chem. Soc. (1950) 1062; J. D. Benigni, R. L. Minnis, J. Heterocycl. Chem. 2 (1965) 387 und DE-A1-37 37 825).

Die Nachteile dieser Verfahren liegen in den niedrigen Ausbeuten und aufwendigen Reinigungsverfahren oder aber in aufwendigen Synthesen und hohen Preisen der Ausgangsmaterialien.

Es besteht daher Bedarf an einem verbesserten Verfahren zur Herstellung der empfindlichen 5,6-Dihydroxyindolderivate, wobei die Produkte aus einem gut zugänglichen Vorläufer in einer glatt ablaufenden Reaktion in hoher Ausbeute und Reinheit erhalten werden sollen.

Überraschenderweise wurde nun gefunden, daß sich diese 5,6-Dihydroxyindole in einfacher Weise dadurch herstellen lassen, daß man die entsprechenden gut zugänglichen Indoline dehydriert.

Gegenstand der Erfindung ist daher ein Verfahren zur Herstellung von 5,6-Dihydroxyindolen der allgemeinen Formel I 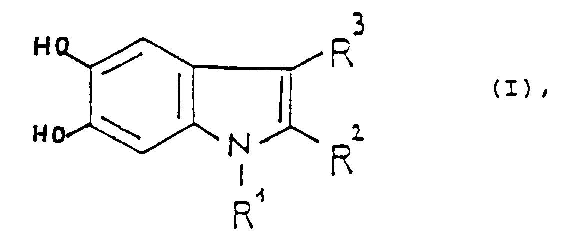 worin R¹ und R³ unabhängig voneinander Wasserstoff oder Alkylgruppen mit 1 bis 4 C-Atomen bedeuten und R² für Wasserstoff, eine Methyl- oder eine Carboxylgruppe steht, durch Dehydrierung von 5,6-Dihydroxyindolinen der Formel II 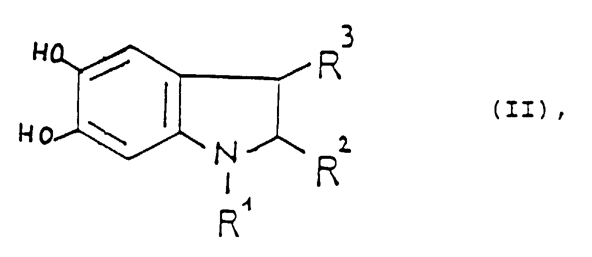 wobei R¹, R² und R³ in Formel II die gleiche Bedeutung wie in Formel I haben, oder deren Salzen.

Bevorzugt wird nach diesem Verfahren 5,6-Dihydroxyindol selbst hergestellt, das als Schlüsselbaustein in der Melaninbiosynthese anzusehen ist und deshalb auch als "natumahes" Vorprodukt zur Erzielung von Oxidationshaarfärbungen verwendet wird.

Die Indoline der Formel II werden entweder in freier Form oder, soweit sie zur Salzbildung befähigt sind, bevorzugt in Form ihrer wasserlöslichen organischen oder anorganischen Salze, insbesondere der Hydrobromide oder Hydrochloride, eingesetzt.

Der Begriff "Dehydrierung" im Sinne der Erfindung soll lediglich zum Ausdruck bringen, daß die 5,6-Dihydroxyindolderivate der Formel I formal durch den Verlust von 2 Wasserstoffatomen aus den entsprechenden Indolinderivaten der Formel II entstehen. Der Begriff "Dehydrierung" soll demnach kein einschränkender Begriff hinsichtlich des Reaktionsmechanismus' oder der für die erfindungsgemäße Dehydrierung zu verwendenden chemischen Mittel sein.

Im Sinne der Erfindung können hierfür alle bekannten Oxidationsmittel, wie z. B. Sauerstoff, Natriumborat, Wasserstoffperoxid und auch die für ihre dehydrierende Wirkung bekannten Mittel wie z. B. Chloranil, N-Bromsuccinimid, Schwefel oder Selen verwendet werden.

Bevorzugt ist jedoch eine katalytische Dehydrierung. Als Dehydrierungskatalysatoren können alle hierfür bekannten Mittel eingesetzt werden, z. B. die Oxide von Chrom, Palladium, Molybdän oder Zink. Vorzugsweise werden jedoch Nickel-, Platin- oder Palladium-haltige Katalysatoren eingesetzt.

Ein weiterer Erfindungsgegenstand ist deshalb das Verfahren, wobei die Dehydrierung in Gegenwart von Dehydrierungskatalysatoren, vorzugsweise Nickel, Platin- oder Palladium-haltigen Katalysatoren, durchgeführt wird.
Solche Katalysatoren sind z. B. feinverteiltes Palladium oder Platin auf Kohle, wobei der Edelmetallgehalt zwischen 3 und 10 Gew.-% liegt oder Raney-Nickel Ganz besonders eignet sich Palladium auf Kohle.

Bevorzugt wird ein einheitlicher Dehydrierungskatalysator eingesetzt. Die Verwendung einer Mischung verschiedener Dehydrierungskatalysatoren ist jedoch im Prinzip möglich.

Als Lösungsmittel können alle für katalytische Dehydrierungen üblichen Lösungsmittel verwendet werden, vorzugsweise wird die Dehydrierung jedoch in Wasser als Lösungsmittel durchgeführt, vor allem dann, wenn dem erfindungsgemäßen Verfahren gemäß 5,6-Dihydroxyindole hergestellt werden sollen. Der Vorteil bei der Verwendung von Wasser als Lösungsmittel liegt darin, daß sich in dem der Reaktion folgenden Extraktionsschritt die Indole der Formel I, selektiv ohne Nebenprodukte mit einem mit Wasser nicht mischbaren organischen Lösungsmittel aus der wäßrigen Phase herausextrahieren lassen und so in hoher Reinheit und Ausbeute erhalten werden können.

Der pH-Wert der wäßrigen Lösung kann dabei zwischen 3 und 11 liegen, vorzugsweise liegt er zwischen 4 und 9.

Vorteilhaft ist der stöchiometrische Zusatz von Wasserstoffakzeptoren, wie z. B. Fumarsäure oder Maleinsäure; diese werden in Form ihrer wasserlöslichen Alkalisalze der Reaktionsmischung zugesetzt.

Das folgende Beispiel dient der Erläuterung der Erfindung und ist nicht einschränkend zu verstehen.

Der pH-Wert der wäßrigen Lösung kann dabei zwischen 3 und 11 liegen, vorzugsweise liegt er zwischen 4 und 9.

Vorteilhaft ist der stöchiometrische Zusatz von Wasserstoffakzeptoren, wie z. B. Fumarsäure oder Maleinsäure; diese werden in Form ihrer wasserlöslichen Alkalisalze der Reaktionsmischung zugesetzt.

Das folgende Beispiel dient der Erläuterung der Erfindung und ist nicht einschränkend zu verstehen.

### Beispiele

### Beispiel 1:

20 g 5,6-Dihydroxyindolin-hydrobromid (86 mMol) wurden in 500 ml Wasser gelöst und nacheinander mit 14 g Fumarsäure-dinatriumsalz (87 mMol) und 1,72 g NaOH (43 mMol) versetzt. Nach Zugabe von 4 g 5 % Pd/C wurde unter Inertgas eine Stunde lang unter Rückfluß gekocht.
Nach Filtration der Reaktionsmischung wurde eine Stunde lang kontinuierlich mit t-Butylmethylether extrahiert. Nach Trocknen der organischen Phase über Magnesiumsulfat und anschließender Filtration wurde das Lösungsmittel entfernt.

Man erhielt 9,8 g (66 mMol) 5,6-Dihydroxyindol als helles, beigefarbenes Pulver, was einer Ausbeute von 76,5 % bezüglich des eingesetzten 5,6-Dihydroxyindolin-hydrobromids entspricht. Eine HPLC-Untersuchung zeigte, daß das so gewonnene 5,6-Dihydroxyindol eine Reinheit von etwa 98 % besitzt.
¹H-NMR (250 MHz, DMSO-d₆):
6,12 ppm (d, J = 3 Hz, 1 H)
6,75 ppm (s, 1 H);
6,82 ppm (s, 1 H);
6,98 ppm (d, J = 3 Hz, 1 H);
Bestimmung der Kopplungskonstanten in MeOH-d₄.

### Beispiel 2:

5 g 5,6 Dihydroxyindolin-hydrobromid (22 mmol) und 860 mg Na0H (22 mmol) wurden in 500 ml H₂O gelöst und in Schutzgasatmosphäre unter Rühren mit 4,92 g Ammoniumperoxodisulfat (22 mmol) versetzt. Nach einer Stunde Rühren bei 25°C wurde filtriert. Im Anschluß daran wurde mit t-Butylmethylether kontinuierlich extrahiert und die so erhaltene Lösung bis zur Trockene eingeengt.

Man erhielt 1,8 g 5,6-Dihydroxyindol (56 % Ausbeute bezüglich 5,6-Dihydroxyindolin-hydrobromid).

## Patentansprüche

1. Verfahren zur Herstellung von 5,6-Dihydroxyindolen der allgemeinen Formel I 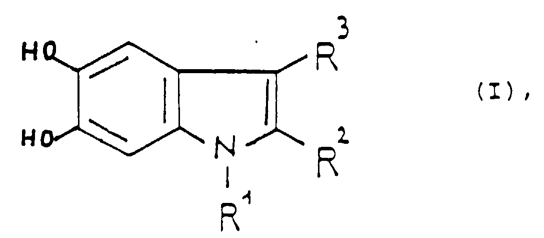 worin R¹ und R³ unabhängig voneinander Wasserstoff oder Alkylgruppen mit 1 bis 4 C-Atomen, R² Wasserstoff, eine Methyl- oder eine Carboxylgruppe bedeuten, durch Dehydrierung von 5,6-Dihydroxyindolinen der Formel II 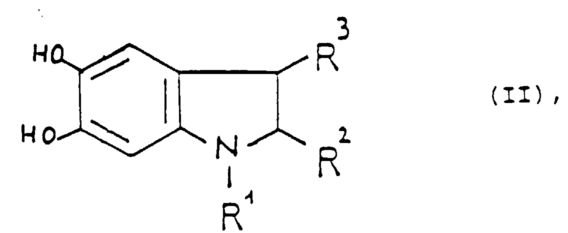 wobei R¹, R² und R³ in Formel II die gleiche Bedeutung wie in Formel I haben, oder deren Salzen.

2. Verfahren nach Anspruch 1 zur Herstellung von 5,6-Dihydroxyindol.

3. Verfahren nach Anspruch 1 und 2, wobei die Dehydrierung in Gegenwart von Dehydrierungskatalysatoren, vorzugsweise Nickel-, Platin- oder Palladium-haltigen Katalysatoren, durchgeführt wird.

## Claims

1. A process for the production of 5,6-dihydroxyindoles corresponding to general formula I: 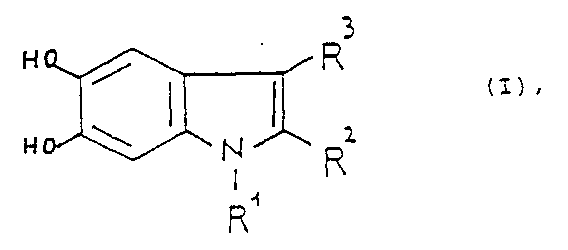 in which R¹ and R³ independently of one another represent hydrogen or alkyl groups containing 1 to 4 carbon atoms, R² is hydrogen, a methyl group or a carboxyl group, by dehydrogenation of 5,6-dihydroxyindolines corresponding to formula II: 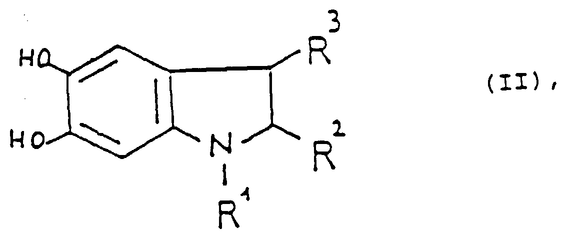 in which R¹, R² and R³ have the same meaning as in formula I,
or salts thereof.

2. A process as claimed in claim 1 for the production of 5,6-dihydroxyindole.

3. A process as claimed in claims 1 and 2, characterized in that the dehydrogenation is carried out in the presence of dehydrogenation catalysts, preferably nickel-, platinum- or palladium-containing catalysts.

## Revendications

1. Procédé pour la préparation de 5,6-dihydroxyindoles répondant à la formule générale I 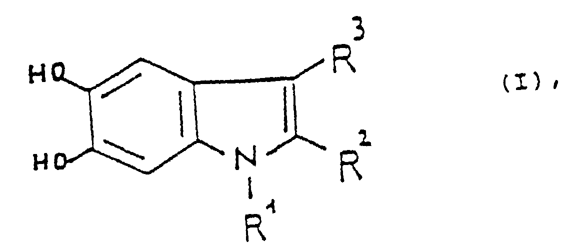 dans laquelle R¹ et R³ représentent, indépendamment l'un de l'autre, un atome d'hydrogène ou des groupes alkyle contenant de 1 à 4 atomes de carbone, R² représente un atome d'hydrogène, un groupe méthyle ou un groupe carboxyle, par déshydrogénation de 5,6-dihydroxyindolines de formule II 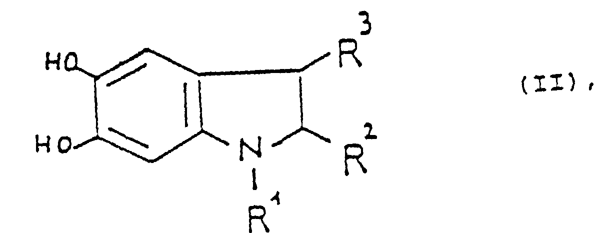 dans laquelle R¹, R² et R³ dans la formule II ont la même signification que dans la formule I, ou de leurs sels.

2. Procédé selon la revendication 1 pour la préparation du 5,6-dihydroxyindole.

3. Procédé selon les revendications 1 et 2, dans lequel on effectue la déshydrogénation en présence de catalyseurs de déshydrogénation, de préférence de catalyseurs contenant du nickel, du platine ou du palladium.
